# EUROPEAN PATENT APPLICATION

(11) **EP 2 154 226 A1**
(43) Date of publication of application: **17.02.2010**
(21) Application number: 08160904.2
(22) Date of filing: 22.07.2008
(51) Int. Cl.: C10L 1/19, C10L 10/12, C10L 1/02, C07C 67/03, C11C 3/10

(54) **Process for manufacturing acid esters through reactive distillation**

(71) Applicant: Yellow Diesel B.V., 1018 WB Amsterdam (NL)
(72) Inventor: Dimian, Alexandre C., 1183 EC Amstelveen (NL); Rothenberg, Gadi, 1096 HS Amsterdam (NL)
(74) Representative: van Looijengoed, Ferry Antoin Theodorus

(57) **Abstract**

The present invention pertains to a process for the manufacture of acid esters through reactive distillation, wherein a carboxylic acid is submitted to esterification in a reaction section of a reactive distillation column with an alcohol in the presence of a solid acid catalyst to form an acid ester, wherein a first supply stream comprising the carboxylic acid, a second supply stream comprising a first alcohol and a third supply stream comprising a second alcohol, the second alcohol having a higher molecular weight than the first alcohol, are supplied to the reactive distillation column, wherein the first supply stream is supplied to the column at a first entry level located just above or at the top of the reaction section, the second supply stream is supplied to the column at a second entry level located in or just below the reaction section and below the first entry level, and the third supply stream is supplied to the column at a third entry level located above the first entry level, and wherein a bottom stream comprising the ester formed is obtained and a top stream comprising unreacted first alcohol, water and unreacted second alcohol is obtained.

The process is particularly suitable for the manufacture of biodiesel from a fatty acid rich feedstock, in particular a feedstock which comprises C10-C18 fatty acids and triglycerides of C10-C18 fatty acid.

## Description

The present invention pertains to a process for manufacturing acid esters through reactive distillation. The process is particularly suitable for the manufacture of biodiesel from waste lipid materials by employing a solid catalyst in a reactive distillation process. In one embodiment a multi-segment tubular liquid-phase reactor is used in combination with a reactive distillation unit.

Biodiesel is a sustainable and renewable fuel produced from various oils, fats and free fatty acids. Conventional feedstock include vegetable and animal lipid materials, more specifically frying and cooking fats (henceforth referred to as "waste fats"), vegetable edible and non-edible oils, animal fats from food processing, industrial greases and solvents, tall oil resulting from kraft pulping and other new renewable sources, such as oil from algae.

In the first step, these materials are reacted with alkyl alcohols. The resulting product is a mixture of fatty acid esters whose application properties as fuel is similar to the conventional petrodiesel fuel obtained from petroleum. Today for the most part the biodiesel is manufactured by the transesterification reaction of glycerides with mono-alcohols. In this reaction, one molecule of triglyceride reacts with one, two, or three molecules of mono-alcohol (most commonly methanol), producing one, two, or three molecules of mono-alkyl fatty esters and water, respectively. Current biodiesel processes typically use an excess of alcohol to shift the chemical equilibrium, increase the rate of the forward reaction, and help fat solubilisation in the liquid mixture. The transesterification is typically catalysed by a homogeneous base catalyst. NaOH, KOH and their corresponding alkoxides are the most used, as described by B. Gutsche in Fett/Lipid 99 (12), 418 (1997). The manufacturing of biodiesel employing heterogeneous base catalysts has been successfully implemented as described in EP-A-1 352 893, US patent 5,908,946 and US patent 6,147,196. Note that using base catalysts requires a feedstock with low free fatty acid (FFA) content, typically below 1% FFA, but preferably below 0.5% FFA, as well as low water content, typically below 500 ppm. The above conditions can be met only by some edible virgin oils. Conversely, waste oil and fat contain typically between 4% FFA and 20% FFA. Consequently, a pre-esterification step is necessary, which typically uses an acid catalyst. Thus, biodiesel manufacturing from waste oil or fat must employ two catalysts, an acid for the esterification pre-treatment, and a base for the transesterification stage.

Another route to the above-mentioned mono-alkyl esters is the direct esterification of FFA with mono-alcohols. In this reaction, one molecule of FFA reacts with one molecule of alcohol, producing one molecule of mono-alkyl fatty ester and one molecule of water. This esterification is catalysed by strong acid catalysts. Current biodiesel processes typically use an excess of alcohol to shift the chemical equilibrium and to increase the rate of the forward reaction. Fatty acid waste oils contain variable amounts of FFA, typically between 5% FFA and up to 100% FFA. These oils and fats are available from various sources, including waste frying oils from restaurants and industrial food processing, yellow grease and animal fats, as well as the tall oil obtained by paper milling. The prices of high-FFA oils are typically less than 50% than those of crop oils. Consequently, these are valuable sources for making low-cost biodiesel with the benefits of avoiding environment pollution

An alternative approach to tackle the variability of raw materials is the full hydrolysis of triglycerides to fatty acids, which can be further submitted to esterification, as described further in this invention. A suitable method is subcritical water hydrolysis that can take place at temperatures of 270 °C to 320 °C and pressures of 10 MPa to 20 MPa. The reaction time can be significantly reduced by an autocatalytic effect due to the initial FFA present in the feedstock [Minami, Saka, Fuels, 2479, 2006]. Up to 90% of the triglycerides can be converted to fatty acid in one step.

The Cetane Number (CN) is an important combustion characteristic of a diesel-type fuels. A minimum CN of 49 is required by the norm DIN 14214. It is known that the CN of fatty acid methyl esters (FAME) increases with the chain length, but decreases with the unsaturation in the hydrocarbon chain. The presence of fatty acid esters of long-chain alcohols, such as butanols or 2-ethyl-hexanol (2EH), increases the CN and as result the biodiesel's combustion capability, but does not affect its viscosity. For example 2EH fatty acid ester has a high CN (Kothe at al., Fuel 82, 971, 2003) and should improve also the cloud point (J. Am. Oil. Chem. Soc. 74, 951, 1997). Hence, the fatty esters of longer chain alkyl alcohol improve the quality of biodiesel mixtures originating from highly unsaturated feedstock, as rapeseed, palm, sunflower or soybean oils. Note that 2EH is a low cost byproduct of n-butyraldehyde production.

Typically, esterification is catalysed by strong acids, such as H₂SO₄, HCl, or p-Me(C₆H₄)SO₃H. A fundamental disadvantage of using a homogeneous catalyst is that it must be neutralised, washed and removed from the product. These operations increase both the capital investment and the operational costs, require additional chemicals and generate contaminated water effluents that must submitted to costly treatment. The low legal limit of sulphur impurities allowed in diesel (currently < 20 ppm) also increases separation costs when sulfur-containing homogeneous catalysts such as H₂SO₄ or p-Me(C₆H₄)SO₃H are used.

Heterogeneous catalysts remain active in-situ for longer periods, do not require chemical neutralisation and do not generate harmful waste. These characteristics greatly simplify the technology. When the solid catalyst finally deactivates, it can be regenerated, recycled and reused. In general, solid base catalysts (SBC) are preferred for transesterification and solid acid catalysts (SAC) for esterification.

SAC are suitable for catalysing fatty acid esterification. Acid resins, such as Nafion and Amberlyst, exhibit good activity, but can be employed only at low to moderate temperatures, in general bellow 130 °C. The use of niobic acid was described recently in US patent application 2007/0232817 A1. Metal oxides can be also applied with better results, since allow using higher reaction temperatures, up to 200°C, with higher productivity and better opportunity for heat integration (Kiss, Dimian and Rothenberg, Energy and Fuels, 2008, 22,598). It is noted that the use of heterogeneous catalysts enables performing the reactions at higher temperatures, resulting also in better use of energy from the heat integration viewpoint.

All esterification reactions produce stoichiometric amounts of water. This water must be removed from the reaction vessel, to shift the chemical equilibrium to the products side and ensure high FFA conversion. When a heterogeneous catalyst is used, water removal is necessary to avoid catalyst deactivation. For example, when sulphated zirconia comes is in contact with free water, sulphonic acid groups may be leached from the catalyst (Kiss, Dimian and Rothenberg, Energy and Fuels, 2008, 22,598). One way to solve this problem is by using Reactive Distillation (RD), also known as Catalytic Distillation. Above 100°C, the water formed by the reaction migrates from the liquid reactive phase to the vapour phase, from which it can be continuously removed by condensation and liquid-liquid separation (Dimian, Omota and Bliek, Chem. Eng. Sci., 59, 3175-3185 2003). When the alcohol forms an azeotrope with water, as in the case of n-propanol, this can be broken by using a suitable entrainer (Dimian, Omota and Bliek , Chemical Engineering & Processing, 43, 411-420, 2004).

The use of an excess of alcohol increases the reaction rate and ensures higher flexibility with respect to FFA content in the feedstock. However, the alcohol excess must be recovered and recycled, which in turn implies additional equipment and separation costs. In a reactive distillation environment a hard constraint can arise from the excessive reboiler temperature due to very high boiling points of fatty acid esters. In this case, the dilution of the bottom product with alcohol can lower the reboiler temperature to acceptable values. As a result, an optimisation of the alcohol excess is necessary, that takes into account the alcohol recycle costs while fulfilling the constraints, such as high FFA conversion and adequate operating temperature.

Since both esterification and transesterification reactions are almost neutral with regard to heat of reaction, biodiesel manufacturing does not involve important energy costs due to the chemical reaction steps. The energy costs are mainly due to physical separation processes. Therefore, reducing the number of separation steps (which are mainly water washing and distillation), results in substantial savings. This can be done using heterogeneous catalysts.

Reactive distillation is a suitable technique for biodiesel manufacturing. A reactive distillation column has three zones: a reaction zone where the chemical reactions producing biodiesel take place, and two separation zones, one separation zone at the top, for water removal and alcohol recycling, and one separation zone at the bottom for alcohol separation from fatty ester product. In the case of esterification the key advantage is that the water produced by reaction can be removed continuously from the liquid to the vapour phase by operating the unit at sufficiently high temperatures, in general above 100°C, as discussed above. In the case of transesterification of a feedstock containing triglycerides, the key advantage to the use of a reactive distillation process is the possibility of generating a much larger ratio alcohol/triglyceride than is needed by stoichiometry, which is necessary to ensure homogeneous liquid reaction phase, higher driving force and control of temperature profile.

The problem underlying the present invention is the provision of a reactive distillation process, in particular a reactive distillation process for the manufacture of biodiesel, which can be operated under more economic conditions, and which provides a product with better properties as compared to reactive distillation processes described in the art.

The present invention is directed to a process for the manufacture of carboxylic acid esters through reactive distillation, wherein a carboxylic acid is reacted in a reaction section of a reactive distillation column with an alcohol under esterification conditions in the presence of a catalyst to form an ester, wherein a first supply stream comprising the carboxylic acid, a second supply stream comprising a first alcohol and a third supply stream comprising a second alcohol, the second alcohol having a higher molecular weight than the first alcohol, are supplied to the reactive distillation column, wherein the first supply stream is supplied to the column at a first entry level located just above or at the top of the reaction section, the second supply stream is supplied to the column at a second entry level located in or just below the reaction section and below the first entry level, and the third supply stream is supplied to the column at a third entry level located above the first entry level, and wherein a bottom stream comprising the ester formed is obtained and a top stream comprising unreacted first alcohol, unreacted second alcohol and water is obtained.

The crux of the process according to the invention is that it is a dual esterification process based on the use of both a first and a second alcohol, wherein the second alcohol has a higher molecular weight than the first alcohol. The second alcohol has the combined function of entrainer of water and co-reactant in the esterification and transesterification reactions.

The process according to the invention has various advantages as compared to the prior art processes which do not use the combination of a first and a second alcohol. In the first place, the use of the second alcohol results in the formation of higher-alcohol fatty acid esters which have a favourable effect on the combustion properties of the biodiesel, as their presence enhances the cetane number. Further, by in situ water removal in the process according to the invention, the chemical equilibrium is shifted in such a way that high conversion of fatty acids is obtained, in general at least 85%, preferably at least 90%, more preferably at least 95%. In some embodiments it may be possible to obtain a conversion of at least 97%, at least 98%, or at least 99%. These conversion percentages also apply to any triglycerides present in the feedstock. Additionally, catalyst deactivation is minimised, which leads to a more stable operating process. Further, the process can be carried out at lower pressures than conventional processes. More in particular, the pressure can be decreased from 15-20 bars as used conventionally to, in a preferred range, 2-6 bar. The excess of light alcohol is decreased as compared to conventional processes.

In one embodiment of the process according to the invention the first alcohol is selected from methanol, ethanol, propanols, and mixtures thereof.

The second alcohol is preferably selected from mono-alkyl-alcohols with 4 to 10 carbon atoms, in particular 6 to 8 carbon atoms.

In a preferred embodiment of the present invention the carboxylic acid is selected from C10 to C18 fatty acids, and mixtures thereof. These fatty acids may be derived from lipid material derived from animal fats, vegetable oils, algae oil or mixtures thereof, including recycled fats, oils and solvents. The feedstock contains 1-100wt.% of fatty acids, more preferable 5 to 100 % FFA.

In one embodiment of the present invention the feedstock comprises C10-C18 fatty acids in combination with fatty acid triglycerides. The amount of fatty acid and triglycerides in the feed varies with the source. In general, the feedstock contains 1-100wt.% of fatty acids. In one embodiment, where the feedstock is derived from oil and non-waste fats, the feedstock contains between 1-30 wt.% of C10-C18 fatty acids and 99-70 wt.% of triglycerides of C10-C18 fatty acids. In another embodiment, where the feed is derived from waste fats, the feed generally contains 5-100% of C10-C18 fatty acids and 0-95 wt.% of triglycerides of C10-C18 fatty acids.

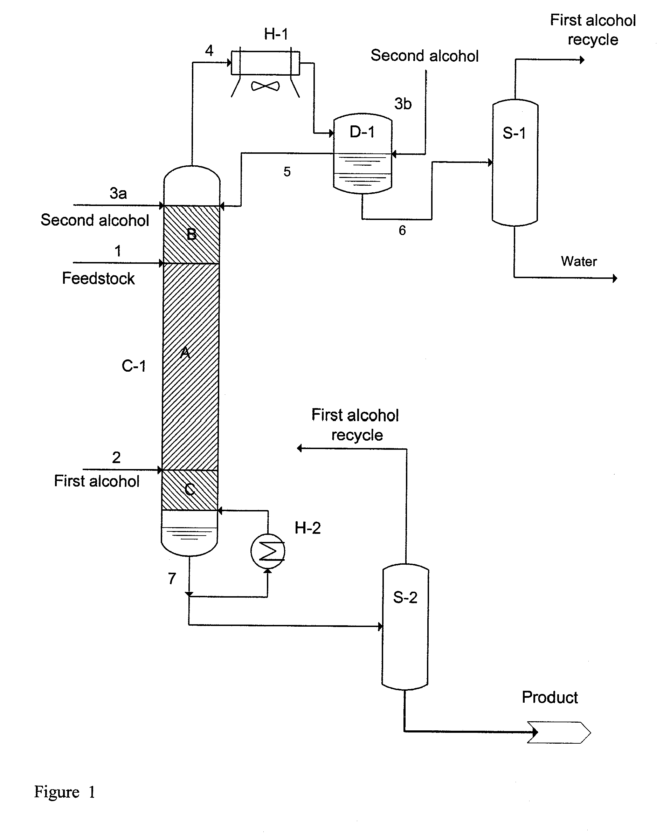

The present invention will be elucidated by way of reference to the figure 1.

In the drawing the reactive distillation column (C-1) comprises a reaction section (A), a separation section (B) and a bottom separation section (C).

A first supply stream comprising the carboxylic acid feedstock, e.g., the FFA feedstock, is fed to the column through a line (1) which enters the column at a first entry level located above or at the top of the reaction section (A).

A second supply stream comprising the first alcohol is fed to the column through a line (2) which enters the column at a second entry level located just below reaction section (A) or directly in the reboiler of the column. As illustrated in figure 1, the second alcohol can be fed to the top of the column (C-1) above the separation section (B) through the line (3a).

A top vapor stream is withdrawn from the top of the reactor through line (4). This top stream comprises unreacted first alcohol, unreacted second alcohol, and water. After condensation in the heat exchanger (H-1), which may be air-cooled or water-cooled, the resulting mixture is submitted to a liquid-liquid separation in the decanting vessel (D-1). It results an organic phase containing first and second alcohols, and an aqueous fraction containing water and sometimes first alcohol. The organic phase is refluxed to the reactive distillation column as the stream (5) to an entry point at the top, or slightly above, the separation section (B). The aqueous phase from the decanter (D-1) is fed through line (6) to a further separation unit (S-1), where first alcohol is separated from water and recycled to the reactive distillation column (C-1). In a preferred embodiment the second alcohol is fed directly into the flash drum (D-1) on level control via the line (3b) entering the column together with the organic phase reflux (6) from the decanter (D-1)) described above. It has been found that this make-up policy allows more robust operation by adapting dynamically the amount of the second alcohol to the consumption by the esterification reaction as well as to the inventory necessary as water entrainer.

The product stream withdrawn from the bottom of the unit (C-1) through line (7) comprises the fatty acid esters, unreacted first alcohol, traces of the second alcohol and traces of water. This stream is subjected to a further processing in a separation unit (S-2), where first alcohol is separated from the product. The light alcohol may be recycled to the column. Het exchanger (H-2) provides energy input to the unit.

The reaction section (A) comprises a solid acid catalyst (SAC) which is effective to convert under esterification conditions the carboxylic acid with the first alcohol and/or the second alcohol to form the resulting esters. Examples of suitable heterogeneous strong acidic catalysts are strong acidic ion-exchange resins as Amberlyte^{™} and Nafion^{™}, acidic ZSM-5 zeolite and acidic beta zeolite, sulphonated or phosphonated inorganic catalysts like sulphonated zirconia or phosphonated zirconia, niobic acid.

The solid catalyst is hosted in a fixed bed arrangement. In one embodiment, the reactive zone consists of structured packing filled with shaped catalyst particles, e.g., catalyst particles shaped as spheres. Other particle shapes may also be used. Examples of suitable packing are Katapack from Sulzer, or Multipack from Monza. Suitable particle size is of 0.8 to 1.2 mm diameter.

The reactive distillation column may have from 10 to 40 equivalent reactive stages, more preferably of 20 to 30 reactive stages, while the number of stages per meter (NSM) can be between one and five, more preferably between two and three.

In addition to the solid acid catalyst, the reaction zone A can also include a solid base catalyst, for carrying out the transesterification reaction to the fatty acid esters. This arrangement is of particular interest when the feedstock contains a relatively high amount of fatty acid triglycerides, e.g., where the feedstock contains 1-50 wt.% of C10-C18 fatty acids and 50-99 wt.% of C10-C18 fatty acid triglycerides, in particular 2-20 wt.% of C10-C18 fatty acids and 80-98 wt.% of C10-C18 fatty acid triglycerides. In this case the in-situ removal of water resulting from the esterification of free fatty acids can be exploited in the sense of protecting de solid base catalyst against deactivation. It is known that the feedstock submitted to transesterification by solid base catalysts should have very low water content, in general less than 1 wt.%, but preferably less than 500 ppm.

Suitable solid base catalysts are known in the art. For example, hydrotalcite or other layered hydroxide compounds may be used. Where a solid base catalyst is present, it is placed in a second reaction zone below the reaction zone containing a solid acid catalyst. This ensures the further conversion of the triglycerides present in the lipid feedstock to monoalkyl fatty esters. The absence of water and fatty acids in the feedstock as it is in contact with the base catalyst greatly helps the preservation of activity of the solid base catalyst.

The base catalyst reactive zone may have from 5 to 30 equivalent reactive stages, more preferably of 5 to 10 reactive stages, while the number of stages per meter (NSM) can be between one and five, more preferably between two and three. As before, in one embodiment the reactive zone consists of structured packing filled with shaped catalyst particles.

The preferred shape of the particles is spheres, of 0.8 mm to 1.2 mm diameter, but other particle shapes and sizes may be used as well. Examples of suitable commercial packing formats are Katapack™ from Sulzer, or Multi-pack™ from Monza.

The first alcohol can be fed entirely at the bottom of the reactive zone, which comprises now both acid solid catalyst and base solid catalyst sections, or partially split to each zone according to reaction requirements.

The reactive distillation column is operated at a pressure between ambient pressure and 20 bar, preferably between 1 and 10 bar, more preferably between 3 and 6 bar. It is noted that the use of a combination of a first and second alcohol allows a significant reduction of the operating pressure required to obtain vapour-liquid equilibrium temperature as compared to the case where only a first alcohol is used. For example, when methanol and 2-ethyl-hexanol are used in the esterification of oleic acid, the required pressure to obtain a temperature of 130°C is 1.5 bar. When only methanol is used, the required pressure is 8 bar.

The operating temperature profile satisfies vapour-liquid equilibrium condition corresponding to the composition of the reactive and non-reactive mixtures. The temperature of the reaction zone may be between 100°C and 220°C, more preferable between 130 and 170°C. The bottom temperature should not exceed 200 °C. This can be controlled by allowing sufficient alcohol in the bottom product.

In general, the preheated feedstock is fed at the top of the reactive reaction section at a temperature between 100-200 °C, more preferably between 130-180 °C.

The first alcohol can be introduced as liquid, as vapour, or as superheated vapour. In one embodiment, the first alcohol is introduced as superheated vapour at the base of the reaction zone. In other embodiment, it is introduced as preheated liquid in the base of the column. The first alcohol feed may contain both fresh feed and recycled alcohol.

The molar ratio of the first alcohol to the carboxylic acid supplied to the column is in the range of from 1:1 to 10:1, preferably in the range of 1:1 to 5:1, more in particular in the range of 1:1 to 2:1.

The molar ratio of the second alcohol to the carboxylic acid supplied to the column is in the range of from 0.1:1 to 1:1, preferably from 0.1:1 to 0.3:1.

Where both a solid acid catalyst and a solid base catalyst are present in the reaction zone, the ratio between the two types of solid catalysts will depend on the feedstock composition, the activity of catalysts, as well as on the target conversion of the transesterification reaction. In general, in this embodiment the catalyst zone will contain 10-90% of solid acid catalyst and 90-10 wt.% of base catalyst. Preferably, the catalyst zone will contain 30-50% of solid acid catalyst and 70-50 wt.% of base catalyst, which should ensure more than 99 % conversion of the FFA and a substantial conversion of triglycerides. Base solid catalyst zone can be extended up to reboiler, inclusive, in order to take advantage from higher temperature.

In some embodiments of the fatty acid ester conversion process of the present invention it may be desired to increase the conversion of triglycerides to esters. This may, for example be the case where the conversion is not complete for kinetic and chemical equilibrium reasons. In this case it may be desired to subject the product of the reactive distillation process to a further transesterification process in an appropriate reactor system with intermediate glycerol removal. Accordingly, in one embodiment the present invention pertains to a process wherein the outlet from the reactive distillation column is submitted to transesterification of glycerides to fatty acid esters in the presence of a solid base catalyst, at a pressure of about 5 to 10 bar above the bubble point pressure of the reaction mixture at the reaction temperature. In a preferred embodiment, this process is carried out in at least two steps, with intermediate removal of glycerol.

The use of tubular reactors for carrying out the transesterification of glycerides in liquid phase with C1-C4 alcohols at has been already described as in EP198243-B1. Ratios 10:1 up to 100:1 are claimed but no details are given about practical implementation. On the other hand the tubular reactor as adiabatic fixed bed column is applied in AXENS/FPI technology, as show in the European patent EP1505048. However, employing fixed bed column reactors is constraint by the limited residence time and hydraulic parameters that cannot cope with the large variability of operating conditions needed when handling waste lipid material.

In one embodiment of the present invention, for overcoming the above limitations this invention makes use of multisegmented tubular reactor with a large ratio length/diameter above 100:1 capable to ensure longer residence time. The tubular reactor is placed in a high temperature chamber, which typically is a furnace heated up by the combustion of gas or liquid fuel.

In a preferred embodiment the reactor consists of a number of equal tubular segments assembled such to shape a reaction coil. The tubular reactor described before has a ratio length/diameter of 100:1 to 1500:1, more preferably of 100:1 to 600:1. The tubes can be placed horizontally or vertically. The initial part of the tube can serve for preheating the reaction mixture up to the reaction temperature. In this way the reactor length can be adapted easily for changing the operating conditions, namely temperature and residence time, imposed by the large variability of the waste lipid materials, as well as to the kinetic property of a particular solid catalyst.

Since getting high conversion, over 95%, and low content of di- and mono-glycerides is limited by the chemical equilibrium, the transesterification reaction is carried out in several stages, usually two, with intermediate glycerol removal. On the other hand, the segregation of a glycerol phase depends heavily on the compositional pattern of the mixture, which in turn is determined by the initial lipid feedstock. Pushing the conversion in the first stage to high values is necessary, over 80% but preferable up to 90%.

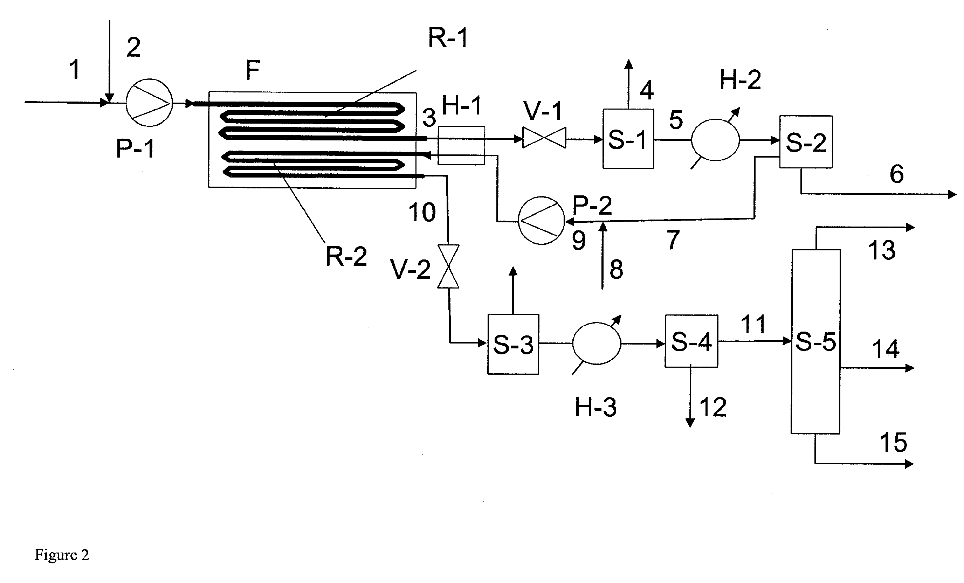

An example of this embodiment of the present invention is illustrated in Figure 2. The bottom product from the reactive distillation section (stream 1) is mixed with first alcohol (stream 2) such to ensure convenient molar ratio alcohol/triglycerides between 3 and 30, more preferably between 9 and 12. The resulting mixture is submitted to pressure raise by the pump (P-1), which should be preferably 5 to 10 bar higher than the corresponding bubble pressure value. Then the resulting mixture enters the tubular reactor (R-1) hosted in the furnace F, where the mixture is brought to reaction temperature. The tubular reactor contains a solid base catalyst. Solid base catalysts are known in the art, and include those mentioned above. The reaction temperature depends on the nature of the catalyst. For an example, the solid base catalyst is a hydrotalcite. In this case the reaction temperature may be, for example, in the range of 150 to 250 °C, more preferably 180 to 210°C. The determination of the appropriate reaction temperature is within the scope of the skilled person.

The outlet mixture from (R-1) is sent to the heat exchanger (H-1) for temperature reduction in counter-current with the cold feed for the second reactor. In his way (H-1) contributes to energy saving. After pressure reduction in (V-1) down to a few bars the mixture is submitted in the unit (S-1)to the partial removal of the light alcohol (stream 4) to facilitate the formation of a glycerol phase. The separation technique can be a simple flash or a sequence of flashes. The liquid phase (stream 5) is cooled in (H-2) to a suitable temperature, typically around 40 C, where phase separation occurs. The separation of glycerol phase (stream 6) takes place in the unit (S-2), by decantation or centrifugation. The stream 7 is submitted to the second stage transesterification in the tubular reactor (R-2), after alcohol make-up (8), pressure raise by the pump (P-2) and preheating in (H-1) with the outlet from (R-1). The transesterification in (R-2) should proceed to more than 95% conversion, preferably to more than 97% conversion in such way that the cumulative content of mono- and di-glycerides becomes lower than 2 wt.%. The effluent from the reactor (R-2) is submitted to pressure reduction in (V-2), deep light alcohol recovery in (S-3), heat exchange in (H-3), glycerol separation in the unit (S-4), glycerol removal through line (12), and purification in the unit (S-5), which is usually a vacuum distillation column. The biodiesel is got as the fatty ester stream (14), while in the top (13) and bottom (15) streams light and heavies impurities are removed.

In this way the combination of the embodiments described in the figures 1 and 2 allows handling any free fatty acids and glycerides feedstock to biodiesel.

It is noted that the base catalyst driven transesterification process carried out using two (or more) tubular reactors in an over is applicable not only to feedstock generated by the reactive distillation process of the present invention, but also to other feedstock containing tri-glycerylesters of fatty acids in combination with fatty acid esters of mono-alcohols.

## Claims

1. A process for the manufacture of acid esters through reactive distillation, wherein a carboxylic acid is submitted to esterification in a reaction section of a reactive distillation column with an alcohol in the presence of a solid acid catalyst to form an acid ester, wherein a first supply stream comprising the carboxylic acid, a second supply stream comprising a first alcohol and a third supply stream comprising a second alcohol, the second alcohol having a higher molecular weight than the first alcohol, are supplied to the reactive distillation column, wherein the first supply stream is supplied to the column at a first entry level located just above or at the top of the reaction section, the second supply stream is supplied to the column at a second entry level located in or just below the reaction section and below the first entry level, and the third supply stream is supplied to the column at a third entry level located above the first entry level, and wherein a bottom stream comprising the ester formed is obtained and a top stream comprising unreacted first alcohol, water and unreacted second alcohol is obtained.

2. A process according to claim 1 wherein the carboxylic acid is a fatty acid, in particular a fatty acid selected from C10-C18 fatty acids and mixtures thereof, and the first supply feed is a fatty acid rich feedstock, in particular a feedstock which comprises C10-C18 fatty acids and triglycerides of C10-C18 fatty acids.

3. A process according to claim 1 or 2, wherein the first alcohol is selected from methanol, ethanol, propanols, and mixtures thereof, more particularly methanol and ethanol.

4. A process according to any one of the preceding claims, wherein the second alcohol is selected from mono-alkylalcohols with 4 to 10 carbon atoms, in particular 6 to 8 carbon atoms, more particularly 2-ethyl-hexanol.

5. A process according to claim 2, wherein the fatty acids are present from 1 to 100 wt.% in a first supply stream which comprises lipid material derived from waste fats, as frying and cooking fats and oils, edible and non-edible vegetable oils, animal fats, tall oil, algae oil, or mixtures thereof, including recycled fats and oils.

6. A process according to any one of the preceding claims, wherein the molar ratio of the first alcohol to the carboxylic acid supplied to the column is in the range of from 1:1 to 10:1, preferably in the range of 1:1 to 5:1, in particular in the range of 1:1 to 2:1 and wherein the molar ratio of the second alcohol to the carboxylic acid supplied to the column is in the range of from 0.1:1 to 1:1, preferably from 0.1:1 to 0.3:1.

7. A process according to any one of the preceding claims, wherein the reaction section comprises a solid acid catalyst, optionally in combination with a solid base catalyst, located below the solid acid catalyst.

8. A process according to any one of the preceding claims wherein the pressure in the reactive distillation column is between ambient pressure and 20 bar, in particular between 2 and 6 bar and the temperature in the reaction section is between 100 and 220°C, in particular between 130 and 180°C.

9. A process according to any one of the preceding claims, wherein the product stream containing the ester formed is subjected to a distillation step to remove unreacted first alcohol, which may be recycled to the distillation unit.

10. A process according to any one of the preceding claims, wherein the top stream product is subjected to a liquid-liquid phase separation by decantation wherein the organic phase containing the first and the second alcohol is separated and recycled to the distillation unit and the water phase separated and sent to waste water treatment or / and first alcohol recovery.

11. A process according to the claim 10 wherein the third supply stream enters the decanter of the reactive distillation column on level control.

12. A process according any one of the preceding claims wherein the outlet from the reactive distillation column is submitted to transesterification of glycerides to fatty acid esters in the presence of a solid base catalyst, at a pressure of about 5 to 10 bar above the bubble point pressure of the reaction mixture at the reaction temperature.

13. A process according to claim 12, wherein the transesterification of glycerides to fatty acid esters takes place in a multi-segment tubular reactor where the length to diameter ratios is 100:1 to 1500:1, more preferably 100:1 to 600:1.

14. A process according to claim 12 or 13 wherein the reaction is carried out in at least two steps with intermediate removal of glycerol.

15. A process according to any one of claims 13 or 14 wherein the tubular reactor is placed in a fired furnace
